**Europäisches Patentamt**

**European Patent Office**

**Office européen des brevets**

(11) Veröffentlichungsnummer : **0 363 521 B1**

(12)

# EUROPÄISCHE PATENTSCHRIFT

(45) Veröffentlichungstag der Patentschrift:
25.09.91 Patentblatt 91/39

(51) Int. Cl.$^5$ : **A61B 3/10**

(21) Anmeldenummer : **88117143.3**

(22) Anmeldetag : **14.10.88**

(54) Einrichtung zur Funktionsprüfung der Otolithen.

(43) Veröffentlichungstag der Anmeldung :
18.04.90 Patentblatt 90/16

(45) Bekanntmachung des Hinweises auf die
Patenterteilung :
25.09.91 Patentblatt 91/39

(84) Benannte Vertragsstaaten :
DE FR GB IT NL SE

(56) Entgegenhaltungen :
EP-A- 0 125 808
DE-A- 3 226 096
US-A- 4 365 874

(73) Patentinhaber : **PANARES TECHNISCHE
ENTWICKLUNGEN GMBH & CO. BETRIEBS
KG
Veilchenstrasse 12
W-8000 München 21 (DE)**

(72) Erfinder : **Vogel, Hagen
Vogelsbergstrasse 30
W-6500 Mainz (DE)**

(74) Vertreter : **Urner, Peter, Dipl.-Phys. Ing. grad.
c/o ter Meer-Müller-Steinmeister & Partner
Mauerkircherstrasse 45
W-8000 München 80 (DE)**

## Beschreibung

Die Erfindung betrifft eine Einrichtung zur Funktionsprüfung der Otolithen gemäß dem Oberbegriff des Patentanspruchs 1.

Wie allgemein bekannt ist, können die Augen neben linearen Bewegungen, d.h. Bewegungen in horizontaler und vertikaler Richtung, auch Rollungen um die visuelle Achse vollziehen. Dem Vestibularapparat (Gleichgewichtsapparat) wurde schon frühzeitig die Kontrolle der Augenbewegungen und so auch für Torsionen eine entscheidende Rolle zugesprochen. Die linearen Augenbewegungen werden nach allgemeiner Auffassung überwiegend durch die drei Bogengänge des Vestibulums gesteuert, während die Augenrollungen überwiegend von den Makulae-Organen des Otolithenapparats induziert werden. Frühzeitig wurde schon die Bedeutung dieser Augenbewegungen für eine klinisch-diagnostische Beurteilung des Vestibularapparats erkannt. Dabei stellt die Messung der Augengegenrollung einen geeigneten Funktionstest zur Überprüfung der Otolithen dar.

Die Augengegenrollung kann im einfachsten Fall durch seitliches Neigen des Kopfes zur Schulter evoziert werden. Der Otolithenapparat als Lagemesser des Kopfes bezüglich des Gravitationsvektors registriert diese Änderung und führt zu einer teilweisen kompensatorischen Rollung der Augen entgegen der Neigungsrichtung.

Um den Einfluß der Hals- und Nackenrezeptoren auf die Augengegenrollung auszuschalten, wurde bisher ein Kipptisch zur geeigneten Lagerung einer Person verwendet, der bei starrer Kopf-Rumpfhaltung den obengenannten Fehler ausschließt. Für die klinische Anwendung ist ein derartiger Kipptisch aber nicht sehr praktikabel, zumal sich hier zusätzliche Probleme einer sicheren Kopf-Körper-Fixation der Person ergeben.

Es wurde bereits nachgewiesen, daß der Einfluß der Nackenrezeptoren auf den Gesamtbetrag der Augengegenrollung nicht größer als etwa 10% ist, so daß auch eine Messung mit aktiver Kopfneigung als hinreichend genauer Otolithentest bezeichnet werden kann.

Wird der Kopf in einer geeigneten Position gehalten, so bleiben auch die Augen statisch in einer Stellung, die gegenüber der ursprünglichen Augenposition um einen bestimmten Betrag gedreht ist. Diese statische Messung der Augengegenrollung ist gegenüber der dynamischen Augengegenrollung relativ klein, die kurzzeitig während der aktiven Kopfkippung entsteht. Das statische Messen reicht jedoch für einen einwandfreien Otolithentest aus und hat den Vorteil, daß keine Einflüsse von seiten der Bogengänge die Beurteilung der Otolithenfunktion verfälschen.

Eine üblicher Methode zur Bestimmung der Augengegenrollung, wie sie allerdings nur in der Grundlagenforschung angewandt wird, ist die bildanalytische Auswertung, bei der z.B. Video-Aufzeichnungen der Augen im Einzelbildverfahren analysiert und vermessen werden.

Diese Methode ist äußerst zeitaufwendig und kommt daher für Routine-Untersuchungen nicht in Betracht. Außerdem können solche Analysen meistens nur Off-line und nicht in Echtzeit durchgeführt werden. Versuche, die Analyse durch nachgeschaltete, schnelle Rechnersysteme automatisiert und On-line durchzuführen, schlugen bisher fehl. Auch die Beobachtung des Augenhintergrunds (Fundoskopie) ist ein Meßverfahren zur Bestimmung der Augengegenrollung durch eine Bildanalyse. Hier werden lediglich Referenzstrukturen in der Iris durch Gefäße in der Retina ersetzt. Es bleibt jedoch die gleiche, umständliche Bild-zu-Bild-Analyse, wie auch bei Benutzung weicher Kontaktlinsen mit eingefärbten Referenzmarken.

Eine Verbesserung stellt der Einsatz von Kontaktlinsen mit eingelassener Sekundärspule dar. Dabei befinden sich der Kopf und die Kontaktlinsen auf den Bulbi in einem homogenen Magnetfeld zweier Helmholtz-Spulen. Bewegungen und Drehungen der Augen können aufgrund der induzierten Spannung in der Sekundärspule direkt gemessen werden. Ein Nachteil dieser Methode ist jedoch, daß bei Kopfneigungen die relativ großen Helmholtz-Spulen mitgeführt werden müssen.

Darüber hinaus führt die Verwendung von Kontaktlinsen noch zu der generellen Schwierigkeit, daß diese auf dem Tränenfilm der Bulbi schwimmen und eine starre Kopplung zwischen Auge und Linse faktisch nicht möglich ist. Selbst bei individuell angepaßten Kontaktlinsen wurden Verschiebungen von über 10° zwischen Auge und Linse gemessen. Klinisch kämen nur universelle Linsen in Frage, bei denen der Krümmungsradius nicht individuell angepaßt ist. Hier dürfte der Fehler dann aber noch größer sein und eine maximal zulässige Fehlertoleranz von ca. 1° bei der diagnostischen Untersuchung erheblich überschreiten.

Eine einfache Methode zur Messung der Augengegenrollung ist die Verwendung eines retinalen Nachbildes. Hier wird ein strichförmiges Nachbild als fixe Referenzlinie bei aufrechter Kopfhaltung auf der Retina erzeugt, das auch bei willkürlichen und reflektorischen Augenbewegungen die subjektive Raumlage nicht verändert. Wird in der gleichen Abbildungsebene eine drehbare zweite Leuchtlinie dargeboten, so kommt es bei Kopfneigung zu einer Drehung zwischen Nachbild und Leuchtlinie, die exakt dem Betrag der statischen Augengegenrollung entspricht. Wird von der zu untersuchenden Person bei weiterhin geneigtem Kopf die Leuchtlinie gedreht und mit dem Nachbild zur Deckung gebracht, so kann auf diese Weise der Betrag der Augengegenrollung ermittelt werden.

Aus US-A-4365874 ist ein Gerät bekannt, das auf Grundlage dieser Methode die Augengegenrollung bestimmt.

Der Erfindung liegt die Aufgabe zugrunde, eine Einrichtung zur Funktionsprüfung der Otolithen zu schaffen, bei der ein retinales Nachbild und eine Leuchtlinie verwendet werden, wobei die Einrichtung leicht handhabbar, kompakt und kostengünstig herstellbar sein soll. Sie soll darüber hinaus eine exakte Ermittlung und schnelle Auswertung der ermittelten Augengegenrollung ermöglichen. Ermittlung und Auswertung sollen dabei elektronisch erfolgen.

Die Lösung der gestellten Aufgabe ist im kennzeichnenden Teil des Patentanspruchs 1 angegeben. Vorteilhafte Ausgestaltungen der Erfindung sind den Unteransprüchen zu entnehmen.

Eine Einrichtung nach der Erfindung zur Funktionsprüfung der Otolithen zeichnet sich aus durch

— ein auf den Kopf einer Person aufsetzbares, abgedunkeltes Brillengestell,

— einen am Brillengestell befestigbaren Neigungssensor, der die Neigung des Kopfes zur linken oder rechten Schulter der Person gegenüber der Vertikalen mißt und ein dem Neigungswinkel entsprechendes Neigungswinkelsignal ausgibt, und

— einen am Brillengestell und vor einem Auge der Person befestigbaren Meßeinsatz, durch den über einen Spalt mittels einer intensitätsstarken Strahlung ein strichförmiges Nachbild auf der Retina sowie bei Neigung des Kopfes über denselben Spalt eine Leuchtlinie mittels einer intensitätsschwachen Strahlung erzeugbar sind, und der eine Einrichtung zum Drehen des Spalts in der Spaltebene sowie eine Meßeinrichtung aufweist, um die Drehung des Spalts, ausgehend von einer Referenzposition, zu messen und ein dem Drehwinkel entsprechendes Drehwinkelsignal auszugeben.

Das Brillengestell kann als maskenartiges Gestell ausgebildet sein und eng am Kopf der Person anliegen. Die Person kann daher mit jeweils einem Auge nur den Spalt des Meßeinsatzes beobachten. Das andere Auge ist abgedeckt.

Vorzugsweise enthält die Einrichtung weiterhin

— eine elektronische Datenverarbeitungseinrichtung, mit der der Neigungssensor und die Meßeinrichtung elektrisch verbunden sind,

— eine mit der Datenverarbeitungseinrichtung verbundene Anzeigeeinrichtung zum Anzeigen des Neigungswinkels und des Drehwinkels, und

— eine Schalteinrichtung, bei deren Betätigung die Datenverarbeitungseinrichtung den angezeigten Neigungswinkel und Drehwinkel zur Durchführung einer Analyse übernimmt.

Bei der Funktionsprüfung der Otolithen einer Person wird zunächst die Augengegenrollung bzw. der ihr entsprechende Drehwinkel bei Neigung des Kopfes nach ein und derselben Seite unter wenigstens zwei verschiedenen Neigungswinkeln $\theta$ gemessen. Dann wird für jeweils einen Neigungswinkel $\theta$ das Verhältnis aus Drehwinkel und Neigungswinkel gebildet. Dieses Verhältnis, das auch als Gain bezeichnet wird, läßt sich dann über den Neigungswinkel $\theta$ auftragen. Im allgemeinen wird eine Gerade erhalten. Bei funktionsfähigen Otolithen liegt diese Gerade innerhalb eines zulässigen Toleranzbereichs. Fällt die Gerade aus dem Toleranzbereich heraus, so liegt ein abnormales Verhalten der Otolithen bzw. des Vestibularapparats vor.

Es ist daher wichtig, die Wertepaare Neigungswinkel und Drehwinkel zur Durchführung einer Analyse gleichzeitig zu übernehmen, was durch die Schalteinrichtung gewährleistet ist.

Um die Augengegenrollung beider Augen einer Person messen zu können, sind der Neigungssensor und der Meßeinsatz wechselweise vor verschiedenen Augen der Person positionierbar. Vorzugsweise sind die ansonsten üblichen Glasplatten im Brillengestell durch Metallplatten mit kreisrunden Öffnungen ersetzt, an deren Umfangsrand sich ein Teil eines Bajonettverschlusses befindet. Der andere Teil ist am Neigungssensor bzw. Meßeinsatz vorhanden. Mit Hilfe des Bajonettverschlusses lassen sich Neigungssensor und Meßeinsatz leicht vertauschen.

Zum Meßeinsatz gehört ein Tubus, der an dem dem Auge der Person zugewandten Ende eine Linsenoptik und am gegenüberliegenden Ende eine Verstellkappe aufweist, die an ihrer Innenseite mit einer koaxial zum Tubus verlaufenden Achse verbunden ist, an deren freiem Ende eine den Spalt aufweisende Beleuchtungseinheit befestigt ist.

Die Person ist somit in der Lage, durch Drehung der Verstellkappe den Spalt und damit die Leuchtlinie so zu verdrehen, daß diese mit dem Nachbild auf der Retina zur Deckung kommt. Bestätigt die Person, daß sich Leuchtlinie und Nachbild decken, so kann z.B. ein Dritter oder die Person selbst die Schalteinrichtung der Datenverarbeitungseinrichtung betätigen, um Neigungswinkel und Drehwinkel zu übernehmen.

Die Beleuchtungseinheit enthält zwei in ihrem Inneren angeordnete Strahlungsquellen, z.B. eine Blitzröhre und eine Leuchtdiodenzeile, die parallel zum Spalt liegen. Die Blitzröhre dient zur Erzeugung des retinalen Nachbildes, während die Leuchtdiodenzeile zur Erzeugung der Leuchtlinie dient. Vorzugsweise kann die Leuchtlinie durch entsprechende Ansteuerung der Leuchtdioden blinkend dargestellt werden, damit die Person besser beurteilen kann, wann sich die Leuchtlinie mit dem Nachbild deckt.

Die Meßeinrichtung kann z.B. als elektrisches Potentiometer ausgebildet sein, dessen Widerstandskörper die Achse konzentrisch umgibt und diese lagert, wobei an der Achse ein den Widerstandskörper berührender Schleifkontakt befestigt ist, oder aber als elektrooptischer Winkelgeber mit zwei relativ zueinander drehbaren Schlitzscheiben und einer elektrooptischen Sende-Empfangseinrichtung. Die Schlitzscheiben können z.B. radial verlaufende Schlitze aufweisen, wobei die von der Sende-Empfangseinrichtung gelieferten Pulse bei Relativdrehung der Schlitzscheiben zur Bestimmung des Drehwinkels zählbar sind. Die Sende-Empfangseinrichtung kann z.B. aus einer lichtemittierenden Diode und aus einer strahlungsempfindlichen Photodiode bestehen.

Alternativ dazu kann die Meßeinrichtung aber auch ein Wechselstrom-Drehmelder sein.

Im folgenden wird die Erfindung unter Bezugnahme auf die Zeichnung näher beschrieben. Es zeigen :

Fig. 1    unterschiedliche Zustände der Einrichtung nach der Erfindung bei der Funktionsprüfung der Otolithen,

Fig. 2    ein Brillengestell mit einem Neigungssensor und einem Meßeinsatz,

Fig. 3    einen Längsschnitt durch den Meßeinsatz nach Fig. 2, und

Fig. 4    ein Blockschaltbild einer Datenverarbeitungseinrichtung, mit der der Neigungssensor und der Meßeinsatz verbunden sind.

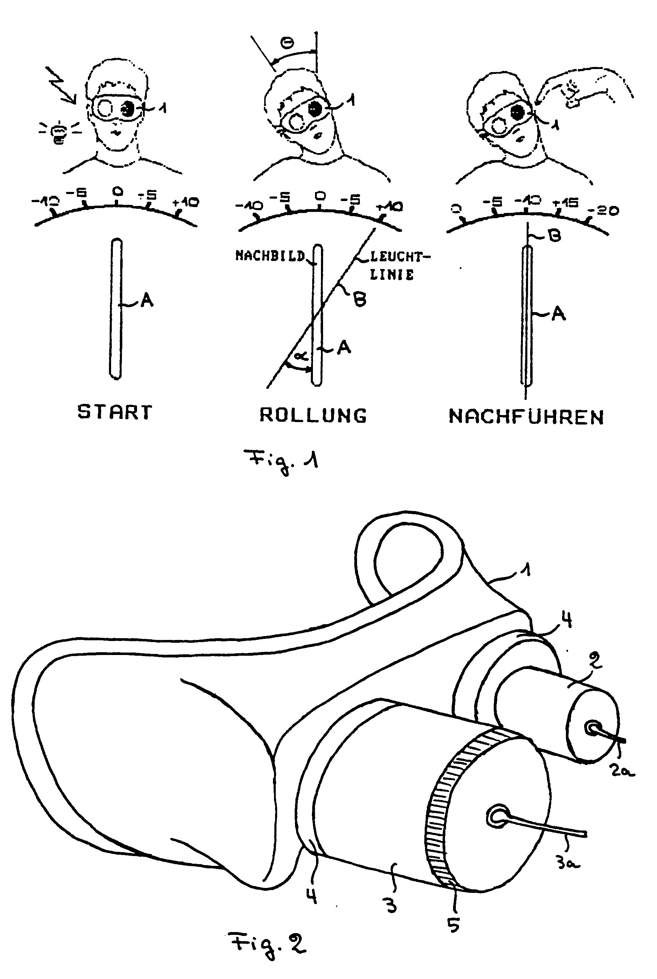

Anhand der Fig. 1 wird zunächst die grundsätzliche Arbeitsweise der Einrichtung zur Funktionsprüfung der Otolithen im einzelnen beschrieben. Mit aufgesetztem Brillengestell 1 bei aufrechter Kopfhaltung wird zunächst in einem ersten Schritt ein strichförmiges Nachbild A als fixe Referenzlinie auf der Retina des Auges erzeugt. Bei geneigtem Kopf wird sodann in derselben Abbildungsebene eine Leuchtlinie B dargeboten, die nunmehr aufgrund der Augengegenrollung gegenüber dem strichförmigen Nachbild A um den Drehwinkel $\alpha$ gedreht ist. Dieser Drehwinkel $\alpha$ entspricht exakt dem Betrag der statischen Augengegenrollung. Wird von der Person durch Drehen der Leuchtlinie B bei weiterhin geneigtem Kopf diese wieder mit dem strichförmigen Nachbild A zur Deckung gebracht, so läßt sich auf diese Weise der Drehwinkel $\alpha$ direkt messen.

Die Fig. 2 zeigt das Brillengestell 1 mit einem Neigungssensor 2 und einem Meßeinsatz 3.

Bei dem Brillengestell 1 kann es sich z.B. um das Gestell einer Taucherbrille handeln, deren Glasscheiben durch Metallplatten mit kreisförmigen Öffnungen ersetzt sind. An den Außenseiten der nicht dargestellten Metallplatten sind der Neigungssensor 2 und der Meßeinsatz 3 über Bajonettverschlüsse 4 angeordnet. Die Bajonettverschlüsse 4 sind so ausgebildet, daß der Neigungssensor 2 und der Meßeinsatz 3 gegeneinander vertauscht werden können. Über Leitungen 2a und 3a sind der Neigungssensor 2 und der Meßeinsatz 3 mit einer noch zu beschreibenden elektronischen Datenverarbeitungseinrichtung verbunden.

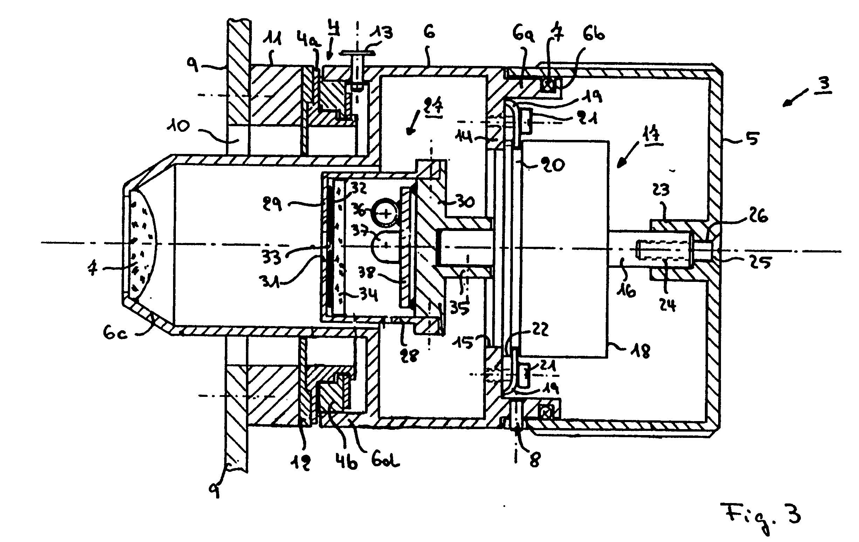

An dem dem Brillengestell abgewandten Ende des Meßeinsatzes 3 ist dieser mit einer Verstellkappe 5 versehen, über die die genannte Person die Leuchtlinie B verdrehen kann. Dies wird nachfolgend unter Bezugnahme auf die Fig. 3 näher beschrieben.

Die Fig. 3 zeigt einen Längsschnitt durch den Meßeinsatz 3 nach Fig. 2. Dieser Meßeinsatz 3 besteht aus einem Tubus 6, der an seinem dem Auge der Person zugewandten Ende eine Linsenoptik 7 trägt. Diese Linsenoptik 7 kann z.B. ein Bikonvexlinse sein. An dem der Linsenoptik 7 gegenüberliegenden Tubusende befindet sich die bereits erwähnte Verstellkappe 5, die koaxial zur Zentralachse des Tubus 6 liegt. Diese Verstellkappe 5 liegt von außen auf einem axialen Ringflansch 6a des Tubus 6 auf und gleitet auf einem Dichtungsring 7, der sich innerhalb einer Umfangsausnehmung 6b des Ringflansches 6a befindet. Ein radialer Stift 8 im axialen Ringflansch 6a dient zur Begrenzung der Bewegung der Verstellkappe 5 in Umfangsdrehrichtung.

Über den bereits erwähnten Bajonettverschluß 4 ist der Tubus 6 bzw. Meßeinsatz 3 mit einer Metallplatte 9 verbindbar, die anstelle eines Brillenglases im Brillengestell 1 vorhanden ist. Statt der Metallplatte 9 kann aber auch eine hinreichend starke und abgedunkelte bzw. lichtundurchlässige Glasplatte verwendet werden. Die Metallplatte 9 weist eine Öffnung 10 auf, durch die das vordere Ende 6c des Tubus 6 hindurchragt, das einen kleineren Außendurchmesser als der Tubus 6 aufweist. Der Innendurchmesser der Öffnung 10 ist größer als der Außendurchmesser des vorderen Endes 6c des Tubus 6, so daß sich der Meßeinsatz 3 senkrecht zu seiner Längsachse relativ zur Metallplatte 9 verschieben läßt, um den interokularen bzw. Augenzwischenabstand genau einstellen zu können.

An der Außenseite der Metallplatte 9 und am Umfangsrand der Öffnung 10 befindet sich ein Tragring 11, der mit der Metallplatte 9 fest verbunden ist. Auf der freien Stirnseite des Tragrings 11 liegt eine ringförmige Scheibe 12, deren Innendurchmesser dem Außendurchmesser des vorderen Endes 6c des Tubus 6 entspricht. Diese ringförmige Scheibe 12 läßt sich ebenfalls parallel zur Metallplatte 9 verschieben, um den Augenzwischenabstand einzustellen. Die ringförmige Scheibe 12 kann nach Einstellen des Augenzwischenabstandes mit dem Tragring 11 durch eine nicht dargestellte Klemmeinrichtung fest verbunden werden. Mit der dem Tragring 11 abgewandten Seite der ringförmigen Scheibe 12 ist ein erster Teil 4a des Bajonettverschlusses 4 fest verbunden. Der zweite Teil 4b des Bajonettverschlusses befindet sich an der Innenseite eines axialen Ringflansches 6d des Tubus 6 und ist mit diesem Ringflansch 6d ebenfalls fest verbunden. Der Ringflansch 6d umgibt dabei konzentrisch das vordere Ende 6c des Tubus 6. Ein Arretierstift 13, der sich in radialer Richtung durch den Ringflansch 6d hindurch erstreckt, dient zur Arretierung bzw. Drehsicherung des Tubus 6 an der Metallplatte 9. Hierzu kann der Arretierstift 13 in eine geeignete Ausnehmung des Teils 4a eingreifen.

Am hinteren Ende des Tubus 6 befindet sich eine Tubus-Innenwand 14 in einer Ebene, die senkrecht zur Längsrichtung des Tubus 6 liegt. Diese Tubus-Innenwand 14 weist eine zentrale Durchgangsöffnung 15 auf, durch die eine drehbare Achse 16 einer Meßeinrichtung 17 hindurchtritt. Die Meßeinrichtung 17 besteht im vorliegenden Ausführungsbeispiel aus einem elektrischen Potentiometer, das einen Widerstandskörper 18 aufweist, der die Achse 16 konzentrisch umgibt und diese Achse 16 ebenfalls lagert. Der Widerstandskörper 18 wird über Klemmen 19 an der Tubus-Innenwand 14 gehalten, und zwar an derjenigen Seite der Tubus-Innenwand 14, die zur Verstellkappe 5 gerichtet ist. Die Klemmen 19 greifen dazu in eine ringförmige Umfangsnut 20 im Widerstandskörper 18 ein und werden mit Hilfe von Schrauben 21, die in die Tubus-Innenwand 14 eingeschraubt werden, in Richtung dieser Wand gedrückt. Dabei wird durch die freien Enden der Klemmen 19 gleichzeitig ein verbleibender Umfangsflansch 22 des Widerstandskörpers 18 gegen die genannte Seite der Tubus-Innenwand 14 gedrückt. Auf diese Weise wird die Meßeinrichtung 17 an der Tubus-Innenwand 14 fixiert.

Die Achse 16 ist durch den Widerstandskörper 18 ebenfalls gegen Verschiebungen in axialer Richtung gesichert. Sie greift mit ihrem einen Ende in einen axialen Ansatz 23 ein, der sich an der Innenseite der Verstellkappe 5 befindet. Die Verstellkappe 5 wird somit auf dem genannten Ende der Achse 16 gelagert. Dieses Achsende besitzt ein axiales Sacklochgewinde 24 zur Aufnahme einer Schraube 25 zur Sicherung der Verstellkappe 5 an der Achse 16, wobei die Schraube 25 eine Bohrung 26 in der Verstellkappe durchragt und mit ihrem Kopf die Verstellkappe 5 gegen das Achsende zieht.

Die genannte Meßeinrichtung 17 dient zur Bestimmung des Drehwinkels $\alpha$ und liefert über eine nicht dargestellte elektrische Leitung ein elektrisches Drehwinkelsignal $S_\alpha$ zu der noch zu beschreibenden Datenverarbeitungseinrichtung. Zu diesem Zweck ist die Achse 16 mit einem nicht dargestellten elektrischen Schleifkontakt verbunden, der den Widerstandskörper 18 beaufschlagt, um das Drehwinkelsignal $S_\alpha$ zu erzeugen.

Das in Richtung der Linsenoptik 7 weisende Ende der Achse 16 trägt auf der anderen Seite der Tubus-Innenwand 14 eine Beleuchtungseinheit 27. Diese Beleuchtungseinheit 27 besteht aus einem hohlzylindrischen Körper 28 mit zwei stirnseitigen Abschlußplatten 29 und 30. Diejenige Abschlußplatte 29, die zur Linsenoptik 7 gerichtet ist, kann unmittelbar einen Spalt aufweisen. Es ist aber auch möglich, in dieser Abschlußplatte 29 einen größeren Schlitz 31 vorzusehen und an der Innenseite der Abschlußplatte 29 eine Spaltblende 32 zur Bildung eines Spalts 33 anzuordnen. An der Innenfläche der Spaltblende 32 befindet sich eine Streuscheibe 34, die den Spalt 33 abdeckt, und durch die zunächst Licht hindurchtritt, bevor es den Spalt 33 durchsetzt.

Die andere und näher an der Achse 16 liegende Abschlußplatte 30 der Beleuchtungseinheit 27 trägt an ihrer äußeren Seite eine einstückig mit ihr verbundene Hülse 35, die auf das freie Ende der Achse 16 aufgesetzt und an diesem drehschlüssig befestigt ist. Die im Inneren der Beleuchtungseinheit 27 liegende Seite der Abschlußplatte 30 trägt zwei Strahlungsquellen 36 und 37, die auf einer Montageplatte 38 angeordnet sind. Die Strahlungsquelle 36 kann eine Blitzröhre sein, die dann gezündet wird, wenn das strichförmige Nachbild A in Fig. 1 auf der Retina des Auges erzeugt werden soll. Diese Blitzröhre ist gegenüber dem Spalt 33 seitlich versetzt und erstreckt sich ferner parallel zur Längsrichtung des Spalts 33. Das Innere der Beleuchtungseinheit 27 wird beim Zünden der Blitzröhre 36 so stark erhellt, daß über die Streuscheibe 34 ein einwandfreies Spaltbild auf der zentralen Achse des Meßeinsatzes 3 erzeugt wird.

Die Strahlungsquelle 37 dient zur Lieferung der intensitätsschwächeren Strahlung und besteht z.B. aus einer Zeile von Leuchtdioden. Die Zeilenrichtung erstreckt sich ebenfalls parallel zur Längsrichtung des Spalts. Dabei liegt die Leuchtdiodenzeile ebenso wie der Spalt auf der zentralen Achse des Meßeinsatzes 3.

Soll das strichförmige Nachbild A auf der Retina des Auges erzeugt werden, so wird zunächst mit Hilfe der Verstellkappe 5 der Spalt 33 in Vertikalstellung ausgerichtet. Dies erfolgt bei aufrechter Kopfhaltung der Person. Danach wird die Blitzröhre 36 kurz gezündet. Nach der Neigung des Kopfes, bei der sich die Spaltstellung relativ zur Linsenoptik 7 nicht verändert, wird mit Hilfe der Leuchtdiodenzeile 37 die Leuchtlinie B erzeugt. Sodann wird die Verstellkappe 5 durch die Person von Hand so verdreht, daß das strichförmige Nachbild A und die Leuchtlinie B zur Deckung kommen. Dabei wird ebenfalls der Schleifkontakt relativ zum Widerstandskörper 18 verdreht, so daß sich eine am Widerstandskörper 18 abgegriffene Spannung verändert. Diese veränderte Spannung wird als Drehwinkelsignal $S_\alpha$ zur Datenverarbeitungseinrichtung übertragen.

Die erforderlichen Steuer- und Versorgungsleitungen für die Strahlungsquellen 36 und 37 sowie für die Meßeinrichtung 17 lassen sich an geeigneter Stelle aus dem Meßeinsatz 3 herausführen und mit der Datenverarbeitungseinrichtung verbinden.

Es sei noch darauf hingewiesen, daß die Linsenoptik 7 relativ zum Spalt 33 in axialer Richtung des Tubus 6 verschoben werden kann, um eine geeignete Fokussierung vorzunehmen. Hierzu kann sich die Linsenoptik 7 z.B. in einer Schraubkappe befinden, die auf das Ende 6c des Tubus 6 aufgeschraubt werden kann.

Obwohl nicht dargestellt, können zwischen dem Spalt 33 und der Linsenoptik 7 zwei parallele Polarisationsfilter angeordnet sein, von denen einer am Tubusende 6c befestigt und der andere über eine Betätigungseinrichtung von Hand in seiner Filterebene verdrehbar ist, wobei die Betätigungseinrichtung an der Außenseite des Meßeinsatzes 3 in einem Bereich vor dem Brillengestell 1 liegt.

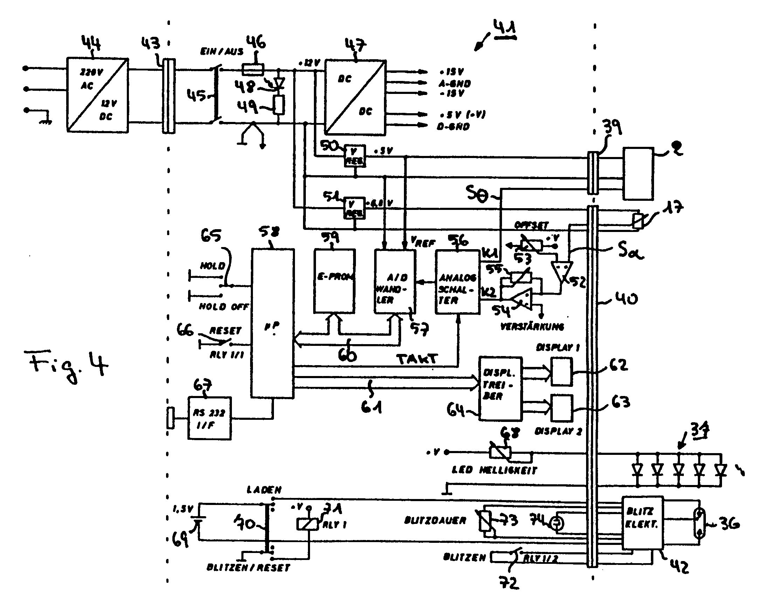

Die Fig. 4 zeigt ein Blockdiagramm der Einrichtung zur Funktionsprüfung der Otolithen. Der Neigungssensor 2 und der Meßeinsatz 3 mit der Meßeinrichtung 17, der Leuchtdiodenzeile 37 sowie der Blitzröhre 36 sind über jeweilige Steckverbindungen 39 und 40 mit einer Datenverarbeitungseinrichtung 41 verbunden. Der Neigungssensor 2 zur Messung der Kopfneigung der Person (Accustar Inklinometer der Firma Schaevitz) arbeitet nach einem kapazitiven Meßverfahren. Er besteht im Prinzip aus einer mit Flüssigkeit und inertem Gas gefüllten, flachen und in der Längsrichtung geteilten Meßzelle. Die Meßzellenhälften sind vertikal mit einem Steg versehen, der zwei Kammern ausbildet. Je nach Neigung des Sensors ändern sich der Flüssigkeitspegel innerhalb der Kammern und damit die Kapazitäten der die Kammerwände bildenden Kondensatoren.

Eine nachgeschaltete, integrierte Elektronik wandelt die Kapazitäten in ein analoges Signal um, das bei Drehung des Sensors in Uhrzeigerrichtung positiv ist und in Gegenuhrzeigerrichtung ein negatives Vorzeichen besitzt. Dieses Signal ist das Neigungswinkelsignal $S_\theta$. Das Null-Signal errechnet sich aus dem 0,5-fachen der Speisespannung. Diese wird in der Datenverarbeitungseinrichtung erzeugt.

Der Meßbereich beträgt ±60° mit einer Linearität von ±0,1°.

Die Meßeinrichtung 17 für die Bestimmung des Drehwinkels α bzw. für die Bestimmung der Augengegenrollung besteht aus einem Präzisionspotentiometer mit ±3% Widerstandstoleranz und ±0,05% Linearitätsfehler, wobei die Versorgung des Präzisionspotentiometers ebenfalls über die Datenverarbeitungseinrichtung 41 erfolgt. Der Meßbereich des Präzisionspotentiometers beträgt im vorliegenden Ausführungsbeispiel ±30°, wobei das vom Präzisionspotentiometer ausgegebene Drehwinkelsignal $S_\alpha$ entsprechend dem Neigungswinkelsignal $S_\theta$ bei Drehung des Präzisionspotentiometers im Uhrzeigersinn positiv und im Gegenuhrzeigersinn negativ ist.

Wie bereits erwähnt, liegt die Blitzröhre 36 zur Erzeugung des retinalen Nachbildes parallel zum Spalt 33 sowie parallel zur Zeile der Leuchtdioden 37. Um keine Hochspannung zwischen der Datenverarbeitungseinrichtung 41 und dem Meßeinsatz 3 übertragen zu müssen, ist die Ansteuerelektronik für die Blitzröhre 36 ebenfalls im Tubus 6 des Meßeinsatzes 3 untergebracht. Diese Ansteuer- bzw. Blitzelektronik ist in Fig. 4 mit dem Bezugszeichen 42 versehen. Sie enthält unter anderem einen nicht dargestellten Ladekondensator zur Durchführung des Blitzbetriebs.

Die Datenverarbeitungseinrichtung 41 ist über einen weiteren Steckverbinder 43 mit einem Netzteil 44 verbunden. Das Netzteil 44 erzeugt aus einer 220V-Eingangswechselspannung eine 12V-Ausgangsgleichspannung und liefert diese über einen Steckverbinder 43 zusammen mit einem Referenzpotential an unterschiedliche Eingänge eines zweipoligen Schalters 45. Die 12V-Gleichspannung wird weiter über eine Sicherung 46 einer Einrichtung 47 zugeführt, die zur Versorgung von analogen Bauteilen der Datenverarbeitungseinrichtung 41 eine Spannung von ± 15 V sowie ein zugeordnetes Referenzpotential ausgibt. Gleichzeitig gibt die Einrichtung 47 eine Spannung von ± 5 V sowie ein zugeordnetes Referenzpotential zur Versorgung digitaler Bausteine der Datenverarbeitungseinrichtung 41 aus. Die Einrichtung 47 empfängt auch das zuvor erwähnte Referenzpotential vom Netzteil 44. Zwischen beiden Eingangsanschlüssen der Einrichtung 47 liegen eine Leuchtdiode 48 und ein mit ihr in Reihe geschalteter Wider stand 49 parallel, um den Betriebszustand anzuzeigen. Das vom Netzteil 44 gelieferte Referenzpotential ist z.B. Erdpotential.

Mit Hilfe von Reglern 50 und 51 werden aus der 12V-Eingangsgleichspannung der Einrichtung 47 stabilisierte Versorgungsspannungen von + 5 V für den Neigungssensor 2 und + 6,8 V für die Meßeinrichtung 17 erzeugt. Beide Regler 50, 51 empfangen auch das Referenzpotential. Dieses wird ebenfalls zum Neigungssensor 2 und zur Meßeinrichtung 17 übertragen. Die entsprechenden Leitungsführungen verlaufen über die genannten Steckverbinder 39 und 40. Die Spannungen von ± 15 V, + 5 V und + 6,8 V sind Gleichspannungen.

Die Datenverarbeitungseinrichtung 41 besitzt zwei Eingangskanäle K1 und K2 mit einem Spannungsbereich von jeweils 0 bis + 5 V. Da das Neigungswinkelsignal $S_\theta$ vom Neigungssensor 2 genau diesem Bereich entspricht, braucht keine weitere Aufbereitung des Neigungswinkelsignals $S_\theta$ zu erfolgen. Eine Feinkalibrierung kann über die Versorgungsspannung vorgenommen werden. Das Neigungswinkelsignal $S_\theta$ wird vom Kanal K1 empfangen.

Das Drehwinkelsignal $S_\alpha$ das die Augengegenrollung angibt, wird dagegen zur besseren Auflösung mit einem Offset überlagert, wobei das verbleibende Nutzsignal auf 5 V für den maximalen Meßbereich von ±30° verstärkt wird. Über die Einstellung des Verstärkungsfaktors kann das Drehwinkelsignal $S_\alpha$ exakt kalibriert werden.

Das Drehwinkelsignal $S_\alpha$ von der Meßeinrichtung 17 wird daher zunächst dem positiven Eingang eines Differenzverstärkers 52 zugeführt, dessen negativer Eingang mit einem Widerstandsabgriff verbunden ist, wobei der zugeordnete Widerstand 53 zwischen einer positiven Spannung und Referenzpotential liegt. Der Ausgang des Differenzverstärkers 52 wird dann einem positiven Eingang eines weiteren Differenz verstärkers 54 (Verstärkung) zugeführt, dessen negativer Eingang ebenfalls das Referenzpotential empfängt. Der Ausgang des zweiten Differenzverstärkers 54 ist einerseits mit dem zweiten Kanal K2 verbunden und andererseits über einen einstellbaren Widerstand 55 zu seinem positiven Eingang zurückgeführt.

Die Eingangskanäle K1 und K2 sind die Eingänge eines Analogschalters 56, der das empfangene Neigungswinkelsignal $S_\theta$ und das Drehwinkelsignal $S_\alpha$ abwechselnd über einen Ausgang zu einem Analog/Digital-Wandler 57 liefert, und zwar in Abhängigkeit eines Taktsignals TAKT, das der Analogschalter 56 an einem weiteren Eingang empfängt. Der Analog/Digital-Wandler 57 weist z.B. eine Auflösung von 10 Bit auf und empfängt weiterhin zur Versorgung das Ausgangssignal des Reglers 50 sowie das Referenzpotential. Die vom Analog/Digital-Wandler 57 ausgegebenen Digitaldaten werden von einem Ein-Chip-Mikroprozessor 58 übernommen. Anhand eines in einem E-PROM 59 gespeicherten Programms erfolgt eine Skalierung der Daten, bei der entsprechende Spannungen bestimmten Winkelgraden zugeordnet werden. Der Analog/Digital-Wandler 57, der Mikroprozessor 58 und der E-PROM 59 sind über einen Datenbus 60 miteinander verbunden. Der Mikroprozessor 58 liefert ebenfalls das Taktsignal TAKT zum Steuereingang des Analogschalters 56.

Über das bereits erwähnte Programm im E-PROM 59 erfolgt auch die Übergabe des Neigungswinkels $\theta$ und des Drehwinkels $\alpha$ vom Mikroprozessor 58 über einen weiteren Datenbus 61 an zwei getrennte 7-Segment-LED-Displays 62 und 63. Diesen Displays 62, 63 ist ein Displaytreiber 64 vorgeschaltet.

Der Mikroprozessor 58 ist ferner mit einer Schalteinrichtung 65, einem Reset-Schalter 66 und einer Schnittstelle 67 (RS 232) verbunden. Hierauf wird später noch genauer eingegangen werden.

Die Leuchtdioden 37 werden über den Steckverbinder 40 mit einer Spannung von + 5 V und mit einem Referenzpotential versorgt. Im spannungsführenden Leiter liegt ein einstellbarer Widerstand 68, über den sich die Intensität der Leuchtdioden 47 einstellen läßt.

Die Versorgung der Ansteuerelektronik 42 der Blitzröhre 36 erfolgt mittels einer 1,5 V-Batterie 69 und ebenfalls über den Steckverbinder 40. Ein doppelpoliger Zweiwegeschalter 70 dient in einer Stellung zum Aufladen des Kondensators in der Ansteuerelektronik 42 und in der anderen Stellung zum Entladen des Kondensators bzw. zum Blitzen der Blitzröhre 36. In der zuletztgenannten Stellung des doppelpoligen Zweiwegeschalters 70 wird ein Relais 71 aktiviert, das dann zwischen einer positiven Versorgungsspannung und Referenzpotential zu liegen kommt. Bei Aktivierung des Relais 71 werden gleichzeitig der Reset-Schalter 66 und ein Blitzschalter 72 geschlossen, dessen beide Pole über den Steckverbinder 40 mit der Ansteuerelektronik 42 verbunden sind. Beim Schließen des Blitzschalters 42 strahlt die Blitzröhre 36 intensitätsstarke Strahlung ab. Das gleichzeitige Schließen des Reset-Schalters 66 bewirkt, daß beim Einschalten der die intensitätsstarke Strahlung aussendenden Blitzröhre 36 gleichzeitig auch der angezeigte Drehwinkel $\alpha$ auf Null zurückgesetzt wird. Auf diese Weise läßt sich der Drehwinkel $\alpha$ bei der nächsten Messung leichter ablesen.

Mit Hilfe eines Potentiometers 73, das über den Steckverbinder 40 mit der Ansteuerelektronik 42 verbunden ist, läßt sich die Blitzdauer der Blitzröhre 36 variieren und damit an die Augenempfindlichkeit der Person anpassen. Die Blitzbereitschaft wird von einer Glimmlampe 74 angezeigt. Die entsprechenden Leitungen zwischen der Glimmlampe 74 und der Ansteuerelektronik 42 sind ebenfalls über den Steckverbinder 40 geführt.

Die Schalteinrichtung 65 arbeitet mit dem Mikroprozessor 58 so zusammen, daß in der Stellung HOLD der Schalteinrichtung 65 die Anzeigewerte beider Displays 62 und 63 eingefroren werden. Der angezeigte Neigungswinkel $\theta$ und der angezeigte Drehwinkel $\alpha$ werden dann nicht mehr verändert. Gleichzeitig können dann in der Stellung HOLD die Meßergebnisse über die RS 232 Schnittstelle 67 einem Rechner zur Auswertung übergeben werden, der z.B. mit einem Monitor oder Drucker zur Darstellung der ausgewerteten Ergebnisse verbunden sein kann. Wird die Schalteinrichtung 65 in die Stellung HOLD OFF gebracht, so werden wieder der aktuelle Neigungswinkel $\theta$ und der aktuelle Drehwinkel $\alpha$ auf den Displays 62 und 63 angezeigt. Die Schalter 65, 70 sowie die Potentiometer 68 und 73 sind von Hand bedienbar.

## Patentansprüche

1. Einrichtung zur Funktionsprüfung von Otolithen, **gekennzeichnet durch**
— ein auf den Kopf einer Person aufsetzbares, abgedunkeltes Brillengestell (1),
— einen am Brillengestell (1) befestigbaren Neigungssensor (2), der die Neigung des Kopfes zur linken oder rechten Schulter der Person gegenüber der Vertikalen mißt und ein dem Neigungswinkel ($\theta$) entsprechendes Neigungswinkelsignal ($S_\theta$) ausgibt, und
— einen am Brillengestell (1) und vor einem Auge der Person befestigbaren Meßeinsatz (3), durch den

EP 0 363 521 B1

über einen Spalt (33) mittels einer intensitätsstarken Strahlung ein strichförmiges Nachbild (A) auf der Retina sowie bei Neigung des Kopfes über denselben Spalt (33) eine Leuchtlinie (B) mittels einer intensitätsschwachen Strahlung erzeugbar sind, und der eine Einrichtung (5, 16, 27) zum Drehen des Spalts (33) in der Spaltebene sowie eine Meßeinrichtung (17) aufweist, um die Drehung des Spalts (33), ausgehend von einer Referenzposition, zu messen und ein dem Drehwinkel entsprechendes Drehwinkelsignal ($S_\alpha$) auszugeben.

2. Einrichtung nach Anspruch 1, **gekennzeichnet durch**
— eine elektronische Datenverarbeitungseinrichtung (41), mit der der Neigungssensor (2) und die Meßeinrichtung (17) elektrisch verbunden sind,
— eine mit der Datenverarbeitungseinrichtung (41) verbundene Anzeigeeinrichtung (62, 63) zum Anzeigen des Neigungswinkels (θ) und des Drehwinkels (α) und
— eine Schalteinrichtung (65), bei deren Betätigung die Datenverarbeitungseinrichtung (41) den angezeigten Neigungswinkel (θ) und Drehwinkel (α) zur Durchführung einer Analyse übernimmt.

3. Einrichtung nach Anspruch 1 oder 2, **dadurch gekennzeichnet**, daß der Neigungssensor (2) und der Meßeinsatz (3) wechselweise vor verschiedenen Augen der Person positionierbar sind.

4. Einrichtung nach Anspruch 1, 2 oder 3, **dadurch gekennzeichnet**, daß zum Meßeinsatz (3) ein Tubus (6, 6a) gehört, der an dem dem Auge der Person zugewandten Ende eine Linsenoptik (7) und am gegenüberliegenden Ende eine Verstellkappe (5) aufweist, die an ihrer Innenseite mit einer koaxial zum Tubus (6) verlaufenden Achse (16) verbunden ist, an deren freiem Ende eine den Spalt (33) aufweisende Beleuchtungseinheit (27) befestigt ist.

5. Einrichtung nach Anspruch 4, **dadurch gekennzeichnet**, daß die Beleuchtungseinheit (27) aus einem hohlzylindrischen Körper (28) mit zwei stirnseitigen Abschlußplatten (29, 30) besteht, von denen diejenige, die der Linsenoptik (7) zugewandt ist, den Spalt (33) aufweist, und die andere im Inneren der Beleuchtungseinheit (27) befindliche Strahlungsquellen (36, 37) zur Erzeugung der intensitätsstarken und der intensitätsschwachen Strahlung trägt.

6. Einrichtung nach Anspruch 5, **dadurch gekennzeichnet**, daß zwischen den Strahlungsquellen (36, 37) und dem Spalt (33) eine Streuscheibe (34) angeordnet ist.

7. Einrichtung nach Anspruch 5 oder 6, **dadurch gekennzeichnet**, daß die Strahlungsquelle (36) zur Erzeugung der intensitätsstarken Strahlung eine Blitzröhre ist, die parallel zum Spalt (33) liegt.

8. Einrichtung nach Anspruch 5, 6 oder 7, **dadurch gekennzeichnet**, daß die Strahlungsquelle (37) zur Erzeugung der intensitätsschwachen Strahlung eine Leuchtdioden-Strahlungsquelle ist.

9. Einrichtung nach Anspruch 8, **dadurch gekennzeichnet**, daß die Leuchtdioden-Strahlungsquelle (37) aus mehreren auf einer Geraden angeordneten Leuchtdioden besteht, die parallel zum Spalt (33) liegt.

10. Einrichtung nach Anspruch 4 oder einem der folgenden, **dadurch gekennzeichnet**, daß die Achse (16) eine Tubus-Innenwand (14) frei durchragt, an der die Meßeinrichtung (17) zur Messung des Drehwinkels (α) des Spalts (33) in Abhängigkeit der Drehstellung der Achse (16) befestigt ist.

11. Einrichtung nach Anspruch 10, **dadurch gekennzeichnet**, daß die Meßeinrichtung (17) ein elektrisches Potentiometer (16, 18) ist, dessen Widerstandskörper (18) die Achse (16) konzentrisch umgibt und diese lagert, an der ein den Widerstandskörper (18) berührender Schleifkontakt befestigt ist.

12. Einrichtung nach Anspruch 10, **dadurch gekennzeichnet**, daß die Meßeinrichtung (17) ein elektrooptischer Winkelgeber mit zwei relativ zueinander drehbaren Schlitzscheiben und einer elektrooptischen Sende-Empfangseinrichtung ist.

13. Einrichtung nach Anspruch 12, **dadurch gekennzeichnet**, daß die von der Sende-Empfangseinrichtung gelieferten Pulse bei Relativdrehung der Schlitzscheiben zur Bestimmung des Drehwinkels (α) zählbar sind.

14. Einrichtung nach Anspruch 10, **dadurch gekennzeichnet**, daß die Meßeinrichtung (17) ein Wechselstrom-Drehmelder ist.

15. Einrichtung nach Anspruch 4 oder einem der folgenden, **dadurch gekennzeichnet**, daß das dem Auge der Person zugewandte vordere Ende (6c) des Tubus (6) einen kleineren Außendurchmesser als der hintere Tubusteil aufweist.

16. Einrichtung nach einem der Ansprüche 4 bis 15, **dadurch gekennzeichnet**, daß am Tubus (6) eine Befestigungseinrichtung (4) zum Befestigen des Meßeinsatzes (3) am Brillengestell (1) vorhanden ist.

17. Einrichtung nach einem der Ansprüche 4 bis 15, **dadurch gekennzeichnet**, daß die Linsenoptik (7) relativ zum Spalt (33) in axialer Richtung des Tubus (6, 6a) verschiebbar ist.

18. Einrichtung nach einem der Ansprüche 5 bis 17, **dadurch gekennzeichnet**, daß die Datenverarbeitungseinrichtung (41) eine Versorgungseinrichtung (50, 51) zur Versorgung von Neigungssensor (2) und Meßeinsatz (3) mit elektrischer Energie sowie von Hand betätigbare Steuereinrichtungen (70, 73, 68) zum Einschalten und Einstellen der Einschaltzeit der intensitätsstarke Strahlung aussendenden Strahlungsquelle

8

(36) bzw. zum Einstellen der Intensität der intensitätsschwache Strahlung aussendenden Strahlungsquelle (37) aufweist.

19. Einrichtung nach Anspruch 18, **dadurch gekennzeichnet**, daß die Steuereinrichtungen zum Einstellen der Einschaltzeit der intensitätsstarke Strahlung aussendenden Strahlungsquelle (36) und zur Einstellung der Intensität der intensitätsschwache Strahlung aussendenden Strahlungsquelle (37) Potentiometer (73, 68) sind.

20. Einrichtung nach Anspruch 1 oder einem der folgenden, **dadurch gekennzeichnet**, daß die Leuchtlinie (B) als blinkende Linie erzeugbar ist.

21. Einrichtung nach Anspruch 4 oder einem der folgenden, **dadurch gekennzeichnet**, daß zwischen dem Spalt (33) und der Linsenoptik (7) zwei parallele Polarisationsfilter angeordnet sind, von denen einer am Tubus (6, 6c) befestigt und der andere über eine Betätigungseinrichtung von Hand in seiner Filterebene verdrehbar ist, wobei die Betätigungseinrichtung an der Außenseite des Meßeinsatzes in einem Bereich vor dem Brillengestell (1) liegt.

22. Einrichtung nach Anspruch 2 oder einem der folgenden, **dadurch gekennzeichnet**, daß die Datenverarbeitungseinrichtung (41) so ausgebildet ist, daß sie bei Einschalten der die intensitätsstarke Strahlung aussendenden Strahlungsquelle (36) gleichzeitig den angezeigten Drehwinkel (α) auf Null zurückstellt.

23. Einrichtung nach Anspruch 2 oder einem der folgenden, **dadurch gekennzeichnet**, daß der Neigungssensor (2) und die Meßeinrichtung (17) elektrisch mit verschiedenen Eingängen (K1, K2) eines Analogschalters (56) verbunden sind, der in Abhängigkeit seines Taktsignals (TAKT) das Neigungswinkelsignal $(S_\theta)$ und das Drehwinkelsignal $(S_\alpha)$ abwechselnd auf seinen Ausgang legt.

24. Einrichtung nach Anspruch 23, **dadurch gekennzeichnet**, daß der Ausgang des Analogschalters (56) über einen Analog/Digitalwandler (57) mit einem Mikroprozessor (58) verbunden ist, der mit einem Nurlesespeicher (59) zusammenarbeitet, in dem eine Spannungen in Winkelgrade umwandelnde Tabelle gespeichert ist.

25. Einrichtung nach Anspruch 2 und 24, **dadurch gekennzeichnet**, daß die Schalteinrichtung (65) mit dem Mikroprozessor (58) verbunden ist, der bei Betätigung der Schalteinrichtung (65) das momentane Neigungswinkelsignal (θ) und Drehwinkelsignal (α) über eine Schnittstelle (67) zu einem Rechner ausgibt.

26. Einrichtung nach Anspruch 23 und 24, **dadurch gekennzeichnet**, daß der Mikroprozessor (58) das Taktsignal (TAKT) liefert.

## Claims

1. Device for testing the operation of otoliths, **characterised by**
— a darkened spectacle frame (1) capable of being worn on the head of a person,
— an inclination sensor (2) capable of being attached to the spectacle frame (1), which inclination sensor (2) measures the inclination of the head towards the left or towards the right shoulder of the person in relation to vertical and emits an angle of inclination signal $(S_\varnothing)$ corresponding to the angle of inclination ($\varnothing$), and
— a measuring inset (3) capable of being attached to the spectacle frame (1) and in front of an eye of the person, by means of which measuring inset (3) an afterimage (A) in lines can be produced on the retina by way of a slot (33) by means of a high intensity emission of rays, and, when the head is inclined, a light line (B) can be produced by way of the same slot (33) by means of a low intensity emission of rays, and which has a device (5, 16, 27) for rotating the slot (33) in the plane of the slot and a measuring device (17) in order to measure the rotation of the slot (33), starting from a reference position, and to emit an angle of rotation signal $(S_\alpha)$ corresponding to the angle of rotation (α).

2. Device according to claim 1, **characterised by**
— an electronic data processing device (41), to which the inclination sensor (2) and the measuring device (17) are electrically connected,
— an indication device (62, 63) connected to the data processing device (41), for indicating the angle of inclination ($\varnothing$) and the angle of rotation (α) and
— a switchgear (65), upon actuation of which the data processing device (41) accepts the indicated angle of inclination ($\varnothing$) and angle of rotation (α) in order to carry out an analysis.

3. Device according to claim 1 or 2, **characterised in that** the inclination sensor (2) and the measuring inset (3) can be positioned alternately in front of different eyes of the person.

4. Device according to claim 1, 2 or 3, **characterised in that**, to the measuring inset (3), there belongs a tube body (6, 6a) which has, at the end facing the eye of the person, an optical lens system (7) and, at the opposite end, an adjustment cap (5) which is connected on the inside to a shaft (16) extending coaxially with the tube body (6), at the free end of which shaft (16) there is attached an illumination unit (27) having the slot (33).

5. Device according to claim 4, **characterised in that** the illumination unit (27) consists of a cylindrical hollow body (28) having two front closing plates (29, 30), of which plates the one facing the optical lens system (7) has the slot (33) and the other one carries radiation sources (36, 37) located inside the illumination unit (27), for generating the high intensity and the low intensity radiation.

6. Device according to claim 5, **characterised in that,** between the radiation sources (36, 37) and the slot (33), a diffusing lens (37) is arranged.

7. Device according to claim 5 or 6, **characterised in that** the radiation source (36) for generating the high intensity radiation is a flash tube which is located parallel to the slot (33).

8. Device according to claim 5, 6 or 7, **characterised in that** the radiation source (37) for generating the low intensity radiation is a light diode radiation source.

9. Device according to claim 8, **characterised in that** the light diode radiation source (37) consists of several light diodes arranged in a straight line located parallel to the slot (33).

10. Device according to claim 4 or one of the subsequent claims, **characterised in that** the shaft (16) projects freely through a tube body internal wall (14) to which there is attached the measuring device (17) for measuring the angle of rotation ($\alpha$) of the slot (33) in dependence upon the rotary position of the shaft (16).

11. Device according to claim 10, **characterised in that** the measuring device (17) is an electrical potentiometer (16, 18), the resistor (18) of which surrounds the shaft (16) concentrically and supports this shaft (16), to which there is attached a sliding contact touching the resistor (18).

12. Device according to claim 10, **characterised in that** the measuring device (17) is an optoelectronic angle transmitter having two slotted discs which can be rotated relative to each other, and an optoelectronic transmitting-receiving device.

12. Device according to claim 12, **characterised in that** the pulses supplied by the transmitting-receiving device can be counted during relative rotation of the slotted discs in order to determine the angle of rotation ($\alpha$).

14. Device according to claim 10, **characterised in that** the measuring device (17) is an a.-c. synchro system.

15. Device according to claim 4 or one of the subsequent claims, **characterised in that** the forward end (6c) of the tube body (6), which end (6c) faces the eye of the person, has a smaller outer diameter than the rear tube body part.

16. Device according to one of claims 4 to 15, **characterised in that,** on the tube body (6), there is an attachment device (4) for the attachment of the measuring inset (3) to the spectacle frame (1).

17. Device according to one of claims 4 to 15, **characterised in that** the optical lens system (7) can be displaced in relation to the slot (33) in the axial direction of the tube body (6, 6a).

18. Device according to one of claims 5 to 17, **characterised in that** the data processing device (41) has a supply device (50, 51) for supplying the inclination sensor (2) and the measuring inset (3) with electrical energy, and hand-operable control devices (70, 73, 68) for switching on and controlling the on-time of the radiation source (36) emitting the high intensity radiation, and for controlling the intensity of the radiation source (37) emitting the low intensity radiation, respectively.

19. Device according to claim 18, **characterised in that** the control devices for controlling the on-time of the radiation source (36) emitting the high intensity radiation and for controlling the intensity of the radiation source (37) emitting the low intensity radiation are potentiometers (73, 68).

20. Device according to claim 1 or one of the subsequent claims, **characterised in that** the light line (B) can be generated as an intermittent line.

21. Device according to claim 4 or one of the subsequent claims, **characterised in that,** between the slot (33) and the optical lens system (7), there are arranged two parallel polarising filters, one of which is attached to the tube body (6, 6c), and the other of which can be rotated in the plane of the filter by hand by way of an operating device, the operating device being located on the outside of the measuring inset in a region in front of the spectacle frame (1).

22. Device according to claim 2 or one of the subsequent claims, **characterised in that** the data processing device (41) is constructed so that, when the radiation source (36) emitting the high intensity radiation is switched on, the said data processing device (41) simultaneously resets the indicated angle of rotation ($\alpha$) to zero.

23. Device according to claim 2 or one of the subsequent claims, **characterised in that** the inclination sensor (2) and the measuring device (17) are electrically connected to different inputs (K1, K2) of an analog-value selector (56) which connects the angle of inclination signal ($S_\emptyset$) and the angle of rotation signal ($S_\alpha$) alternately to its output in dependence upon its clock signal (TAKT).

24. Device according to claim 23, **characterised in that** the output of the analog-value selector (56) is connected by way of an analog-to-digital converter (57) to a microprocessor (58) which cooperates with a read-only memory (59) in which there is stored a table converting voltages to degrees of angle.

25. Device according to claims 2 and 24, **characterised in that** the switchgear (65) is connected to the microprocessor (58) which, when the switchgear (65) is operated, transmits the instantaneous angle of inclination signal ($\varnothing$) and angle of rotation signal ($\alpha$) by way of an interface (67) to a computer.

26. Device according to claims 23 and 24, **characterised in that** the microprocessor (58) supplies the clock signal (TAKT).


## Revendications

1. Dispositif pour contrôler le fonctionnement d'otolithes, caractérisé par
— une monture de lunettes (1) foncée, pouvant être installée sur la tête d'une personne,
— un détecteur d'inclinaison (2), qui peut être fixé sur la monture de lunettes (1), mesure l'inclinaison de la tête sur l'épaule gauche ou l'épaule droite de la personne par rapport à la verticale et délivre un signal d'angle d'inclinaison ($S_\theta$) correspondant à un angle d'inclinaison, et
— un insert de mesure (3), qui peut être fixé sur la monture de lunettes (1), devant un oeil de la personne et qui permet de produire, par l'intermédiaire d'une fente (33) et au moyen d'un rayonnement de forte intensité, une image (A) en forme de trait sur la rétine ainsi que, dans le cas de l'inclinaison de la tête, une ligne lumineuse (B) par l'intermédiaire de la même fente (33) et au moyen d'un rayonnement de faible intensité, et qui comprend un dispositif (5, 16, 27) permettant de faire tourner la fente (33) dans son plan ainsi qu'un dispositif de mesure (17) permettant de mesurer la rotation de la fente (33) à partir d'une position de référence et de délivrer un signal d'angle de rotation ($S_\alpha$) qui correspond à l'angle de rotation ($\alpha$).

2. Dispositif selon la revendication 1, caractérisé par :
— un dispositif électronique de traitement des données (41) auquel sont raccordés électriquement le détecteur d'inclinaison (2) et le dispositif de mesure (17),
— un dispositif d'affichage (62, 63) raccordé au dispositif de traitement de données (41) servant à afficher l'angle d'inclinaison ($\theta$) et l'angle de rotation ($\alpha$), et
— un dispositif de commutation (65), lors de l'actionnement duquel le dispositif de traitement de données (41) reçoit l'angle d'inclinaison ($\theta$) et l'angle de rotation ($\alpha$), indiqués, pour exécuter une analyse.

3. Dispositif selon la revendication 1 ou 2, caractérisé en ce que le détecteur d'inclinaison (2) et l'insert de mesure (3) peuvent être positionnés alternativement devant les yeux différents de la personne.

4. Dispositif selon la revendication 1, 2 ou 3, caractérisé en ce que l'insert de mesure (3) comporte un tube (6, 6a), qui possède, sur l'extrémité tournée vers l'oeil de la personne, un système optique à lentille (7) et, sur l'extrémité opposée, un capuchon de réglage (5), qui est raccordé, au niveau de sa face intérieure, à un axe (16) coaxial au tube (6) et à l'autre extrémité duquel est fixée une unité d'éclairage (27) comportant la fente (33).

5. Dispositif selon la revendication (4), caractérisé en ce que l'unité d'exposition (27) est constituée par un corps cylindrique creux (28) comportant deux plaques frontales de fermeture (29, 30), dont celle, qui est tournée vers le système optique à lentille (7), comporte la fente (33) et dont l'autre porte d'autres sources de rayonnement (36, 37) situées à l'intérieur de l'unité d'exposition (27) et servant à produire le rayonnement de forte intensité et le rayonnement de faible intensité.

6. Dispositif selon la revendication 5, caractérisé en ce qu'un disque diffuseur (34) est disposé entre les sources de rayonnement (36, 37) et la fente (33).

7. Dispositif selon la revendication 5 ou 6, caractérisé en ce que la source de rayonnement (36) servant à produire le rayonnement de forte intensité est un tube éclair, qui est parallèle à la fente (33).

8. Dispositif selon la revendication 5, 6 ou 7, caractérisé en ce que la source de rayonnement (37) servant à produire le rayonnement de faible intensité est une source de rayonnement à diode à luminescence.

9. Dispositif selon la revendication 8, caractérisé en ce que la source de rayonnement à diode à luminescence (37) est constituée par plusieurs diodes à luminescence disposées sur une droite qui est parallèle à la fente (33).

10. Dispositif selon la revendication 4 ou l'une des revendications suivantes, caractérisé en ce que l'axe (16) traverse librement une paroi intérieure (14) du tube, sur laquelle est fixé le dispositif de mesure (10) servant à mesurer l'angle de rotation ($\alpha$) de la fente (33) en fonction de la position en rotation de l'axe (16).

11. Dispositif selon la revendication 10, caractérisé en ce que le dispositif de mesure (17) est un potentiomètre électrique (16, 18), dont le corps formant résistance (18) entoure concentriquement et supporte l'axe (16), sur lequel est fixé un contact glissant qui est en contact avec le corps formant résistance (18).

12. Dispositif selon la revendication 10, caractérisé en ce que le dispositif de mesure (17) est un capteur angulaire électro-optique comportant deux disques fendus pouvant tourner l'un par rapport à l'autre et un dispositif d'émission-réception électro-optique.

13. Dispositif selon la revendication 12, caractérisé en ce que les impulsions délivrées par le dispositif d'émission-réception peuvent être comptées lors de la rotation relative des disques fendus pour la détermination de l'angle de rotation ($\alpha$).

14. Dispositif selon la revendication 10, caractérisé en ce que le dispositif de mesure (17) est un résolveur à courant alternatif.

15. Dispositif selon la revendication 4 ou l'une des suivantes, caractérisé en ce que l'extrémité avant (6c) du tube (6), qui est tournée vers l'oeil de la personne, possède un diamètre extérieur inférieur à la partie arrière du tube.

16. Dispositif selon l'une des revendications 4 à 15, caractérisé en ce que sur le tube (6) est installé un dispositif de fixation (4) servant à fixer l'insert de mesure (3) sur la monture de lunettes (1).

17. Dispositif selon l'une des revendications 4 à 15, caractérisé en ce que le système optique à lentille (7) est déplaçable par rapport à la fente (33) dans la direction axiale du tube (6, 6a).

18. Dispositif selon l'une des revendications 5 à 7, caractérisé en ce que le dispositif de traitement de données (41) comporte un dispositif d'alimentation (50, 51) servant à alimenter le détecteur d'inclinaison (2) et l'insert de mesure (3) avec une énergie électrique, ainsi que des dispositifs de commande (70, 73, 68) pouvant être actionnés manuellement et servant à brancher et régler la durée de branchement de la source de rayonnement (36) émettant le rayonnement de forte intensité ou régler l'intensité de la source de rayonnement (37) émettant le rayonnement de faible intensité.

19. Dispositif selon la revendication 18, caractérisé en ce que le dispositif de commande servant à régler la durée de branchement de la source de rayonnement (36) émettant le rayonnement de forte intensité et à régler l'intensité de la source de rayonnement (37) émettant le rayonnement de faible intensité sont des potentiomètres (73, 68).

20. Dispositif selon la revendication 1, ou l'une des suivantes, caractérisé en ce que la ligne lumineuse (B) peut être produite sous la forme d'une ligne clignotante.

21. Dispositif selon la revendication 4 ou l'une des suivantes, caractérisé en ce qu'entre la fente (33) et le système optique à lentille (7) sont disposés deux filtres de polarisation parallèles, dont l'un est fixé sur le tube (6, 6c), tandis qu'on peut faire tourner manuellement l'autre filtre de polarisation dans son plan à l'aide d'un dispositif d'actionnement, qui est situé sur la face extérieure de l'insert de mesure, dans une zone située devant la monture de lunettes (1).

22. Dispositif selon la revendication 2 ou l'une des suivantes, caractérisé en ce que le dispositif de traitement de données (48) est agencé de telle sorte que lors du branchement de la source de rayonnement (36) émettant le rayonnement de forte intensité, il ramène simultanément à zéro l'angle de rotation ($\alpha$) indiqué.

23. Dispositif selon la revendication 2, ou l'une des suivantes, caractérisé en ce que le détecteur d'inclinaison (2) et le dispositif de mesure (17) sont raccordés électriquement aux différentes entrées (K1, K2) d'un interrupteur analogique (58), qui délivre alternativement à sa sortie le signal d'angle d'inclinaison ($S_\theta$) et le signal d'angle de rotation ($S_\alpha$) en fonction de son signal de cadence (CADENCE).

24. Dispositif selon la revendication 23, caractérisé en ce que la sortie de l'interrupteur analogique (56) est raccordée par l'intermédiaire d'un convertisseur analogique/numérique (57) à un processeur (58) qui coopère avec une mémoire morte (59), dans laquelle est mémorisé un tableau convertissant des tensions en des degrés d'angle.

25. Dispositif selon les revendications 2 et 24, caractérisé en ce que le dispositif de commutation (56) est raccordé au microprocesseur (58), qui, lors de l'actionnement du dispositif de commutation (65), envoie le signal d'angle d'inclinaison (0) et le signal d'angle de rotation ($\alpha$), instantanés, à un ordinateur au moyen d'une interface (67).

26. Dispositif selon les revendications 23 et 24, caractérisé en ce que le microprocesseur (58) délivre le signal de cadence (CADENCE).

**START**   **ROLLUNG**   **NACHFÜHREN**

Fig. 1

Fig. 2

Fig. 3

Fig. 4